# EUROPÄISCHE PATENTSCHRIFT

(11) **EP 2 438 192 B1**
(45) Veröffentlichungstag und Bekanntmachung des Hinweises auf die Patenterteilung: **25.01.2017**
(21) Anmeldenummer: 10726931.8
(22) Anmeldetag: 03.06.2010
(51) Int. Cl.: C12Q 1/68

(54) **AMPLIFIKATIONSKONTROLLE FÜR KOMPLEXER NUKLEINSÄUREN**
AMPLIFICATION CONTROL FOR COMPLEX NUCLEIC ACIDS
CONTRÔLE DE L'AMPLIFICATION D'ACIDES NUCLÉIQUES COMPLEXES

(30) Priorität: 04.06.2009 DE 102009024143
(43) Veröffentlichungstag der Anmeldung: 11.04.2012
(73) Patentinhaber: Qiagen GmbH, 40724 Hilden (DE)
(72) Erfinder: KORFHAGE, Christian, 40764 Langenfeld (DE); FISCH, Evelyn, 40724 Hilden (DE)
(74) Vertreter: CH Kilger Anwaltspartnerschaft mbB
(86) Internationale Anmeldenummer: PCT/EP2010/057793
(87) Internationale Veröffentlichungsnummer: WO 2010/139767

(56) Entgegenhaltungen:
- EP-A1- 0 623 682
- EP-A1- 1 484 394
- EP-A2- 0 714 987
- US-A1- 2003 228 613
- LI NI ET AL: "CE combined with rolling circle amplification for sensitive DNA detection" ELECTROPHORESIS, Bd. 29, Nr. 2, Januar 2008 (2008-01), Seiten 424-432, XP002599316 ISSN: 0173-0835
- TALSETH-PALMER BENTE A ET AL: "Whole genome amplification and its impact on CGH array profiles." BMC RESEARCH NOTES 2008 LNKD- PUBMED:18710509, Bd. 1, 2008, Seite 56, XP002599317 ISSN: 1756-0500
- WANG G ET AL: "DNA amplification method tolerant to sample degradation" GENOME RESEARCH, COLD SPRING HARBOR LABORATORY PRESS, WOODBURY, NY, US LNKD- DOI:10.1101/GR.2813404, Bd. 14, 1. Januar 2004 (2004-01-01), Seiten 2357-2366, XP002391762 ISSN: 1088-9051
- Molecular Probes: "Quant-1TTM PicoGreen ® dsDNA Reagent and Kits" 10. Juni 2008 (2008-06-10), XP002599318 Gefunden im Internet: URL:http://probes.invitrogen.com/media/pis /mp07581.pdf [gefunden am 2010-08-26]

### SEQUENCE LISTING

<110> Qiagen GmbH
<120> Amplifikation komplexer Nukleinsäuren
<130> R1526 PCT BLN
<160> 10
<170> PatentIn version 3.5
<210> 1
   <211> 20
   <212> DNA
   <213> Artificial Sequence
<220>
   <223> Primer
<400> 1
   gagacgctgg agtacaaacg 20
<210> 2
   <211> 22
   <212> DNA
   <213> Artificial Sequence
<220>
   <223> Primer
<400> 2
   ccagcggatt atcgccatac tg 22
<210> 3
   <211> 18
   <212> DNA
   <213> Artificial Sequence
<220>
   <223> Primer
<400> 3
   tgctccctgt cccatctg 18
<210> 4
   <211> 24
   <212> DNA
   <213> Artificial Sequence
<220>
   <223> Primer
<400> 4
   agacagtatg cctttatttc accc 24
<210> 5
   <211> 22
   <212> DNA
   <213> Artificial Sequence
<220>
   <223> Primer
<400> 5
   gtctttagct gctgaggaaa tg 22
<210> 6
   <211> 21
   <212> DNA
   <213> Artificial Sequence
<220>
   <223> Primer
<400> 6
   agcagaattc tgcacatgac g 21
<210> 7
   <211> 21
   <212> DNA
   <213> Artificial Sequence
<220>
   <223> Primer
<400> 7
   agtgctaatg tcatgtctct t 21
<210> 8
   <211> 19
   <212> DNA
   <213> Artificial Sequence
<220>
   <223> Primer
<400> 8
   gctgagagga gtggaagct 19
<210> 9
   <211> 21
   <212> DNA
   <213> Artificial Sequence
<220>
   <223> Primer
<400> 9
   agtgctaatg tcatgtctct t 21
<210> 10
   <211> 19
   <212> DNA
   <213> Artificial Sequence
<220>
   <223> Primer
<400> 10
   gctgagagga gtggaagcc 19

## Beschreibung

### Technisches Gebiet der Erfindung

Die vorliegende Erfindung betrifft das Gebiet der Biologie und Chemie, insbesondere der Molekularbiologie. Im Speziellen betrifft die Erfindung die Amplifikation von komplexer Nukleinsäuren, wie z.B. gesamter Genome, Transkriptome und Bisulfitome

### Hintergrund der Erfindung

Die Amplifikation von komplexen Template-Nukleinsäuren spielt in vielen molekularbiologischen Anwendungen eine wichtige Rolle. So ist es für einige Anwendungen z.B. erforderlich im vollständige Genome (Whole-Genome-Amplifikation, WGA), bzw. vollständige Transkriptome (Whole- Transkriptom-Amplifikation, WTA) oder vollständige Bisulfitome (Whole-Bisulfitome-Amplifikation, WBA) zu amplifizieren.

Für den Erfolg der Amplifikation solcher, komplexer Nukleinsäuren sind verschiedene Parameter von entscheidender Bedeutung:
Notwendige Vorraussetzung sind geeignete Reaktionsbedingungen. Hierzu zählt z.B. ein geeigneter Puffer mit einem geeigneten pH-Wert und mit einer geeigneten Komposition an monovalenten und bivalenten Salzen. Hierzu zählen aber auch mindestens eine geeignete Polymerase und geeignete Reaktionstemperaturen für die Amplifikation sind essentielle Faktoren für den Erfolg der Amplifikation

Ein entscheidender Parameter ist die Menge an komplexer Template-Nukleinsäure, die eingesetzt wird. Ist die Menge an komplexer Template-Nukleinsäure zu klein, so kann z.B. nicht das gesamte Genom abgedeckt werden. Beispielsweise entspricht eine Menge von 6 pg humaner genomischer DNA ungefähr dem haploiden menschlichen Genom. Setzt man weniger als diese 6 pg in eine WGA Reaktion ein, so ist es unmöglich in der WGA Reaktion das humane Genom vollständig zu amplifizieren, da nicht alle Bereiche repräsentiert sind. Auf der anderen Seite kann eine Menge von weniger als 100 pg ebenfalls zu klein sein, da bei einer derartigen Menge bestimmte Bereich aus stochastischen Effekten über- bzw. unterrepräsentiert sind.

Ein dritter entscheidender Parameter für Amplifikation komplexer Template-Nukleinsäuren ist die Qualität der Probe bzw. der darin enthaltenen komplexen Template-Nukleinsäure(n). Unter dem Begriff "Qualität" können verschiedene Aspekte zusammengefasst werden:
Die die zu amplifizierende komplexe Template-Nukleinsäure enthaltende Probe kann Inhibitoren aufweisen, die die verwendete Polymerase kompetitiv oder allosterisch hemmen oder die aktive Konformation der Polymerase zerstören. Solche Substanzen werden im Folgenden *trans-*Inhibitoren genannt. Zu solchen Trans-Inhibitoren zählen z.B. Schweremetallionen (z.B. Ni, Fe, Mn, Zn, etc.), negative geladene Polymere (z.B. Heparin, Dextransulfat etc.) oder Protein-denaturierende Substanzen, die häufig bei Nukleinsäure-Präparationen verwendet werden (z.B. SDS, Phenol etc.).

Die Probe kann aber auch Stoffe enthalten, die an Nukleinsäuren binden und hierdurch die Amplifikation der Nukleinsäure inhibieren, z. B. durch das Verhindern einer Denaturierung der Nukleinsäure oder durch die Verhinderung der Erkennung der Nukleinsäure durch eine Polymerase. Solche Substanzen werden im Folgenden *cis*-Inhibitoren genannt. Solche cis-Inhibitoren können Proteine (z.B. Histone etc.), positiv geladene Agenzien (z.B. positiv geladene Polymere, positiv geladene Aminosäure etc.)

Die Nukleinsäure kann auf irgendeine Art zerstört sein, so dass an den zerstörten Stellen in der Nukleinsäure die Polymerase keine Verlängerungsreaktion durchführen kann. Diese zerstörten Stellen können z. B. Einzelstrang- oder Doppelstrangbrüche oder abasische Stellen sein oder Stellen, an denen veränderte Basen vorliegen (z. B. zu Oxoguanin oder Uracil in DNA).

Diese quantitativen, bzw. qualitativen Merkmale können derzeit nur in unzureichenden Maße zu Beginn einer Reaktion zur Amplifikation komplexer Template-Nukleinsäuren festgestellt werden. Es ist häufig nicht klar, in welcher Qualität oder Menge die zu amplifizierende komplexe Template-Nukleinsäure vorliegt. So kann bei sehr kleinen Mengen an komplexer Template-Nukleinsäure die Konzentrationsmessung nicht durch Messung der OD260 erfolgen. DNA Schäden können nur durch Gelelektrophorese festgestellt werden, wenn die DNA Menge so groß ist, dass eine Elektrophorese durchgeführt werden kann. Andere DNA Schäden, wie z.B. abasische DNA Bereiche können derzeit nicht bestimmt werden.

Eine Kontrolle dieser qualitativen Parameter der komplexen Template-Nukleinsäure über z.B. quantitative PCR ist nur unzureichend möglich, da über quantitative PCR nur schlecht Größendifferenzen bei teilweise degradierter DNA nachgewiesen werden können solange die Template-Nukleinsäure eine durchschnittliche Größe von > 1 kb hat. Solche Größendifferenzen können aber für die Amplifikation einer komplexen Nukleinsäure schon eine entscheide Rolle spielen.

### Beschreibung der Erfindung

Die vorliegende Erfindung betrifft daher ein in Anspruch 1 definiertes Verfahren zur quantitativen und qualitativen Analyse einer oder mehrerer zu amplifizierenden komplexen Template-Nukleinsäure in einer Probe umfassend die folgenden Schritte:
Bereitstellung eines Reaktionsgemisches umfassend,
   - die zu amplifizierende komplexe Template-Nukleinsäure,
   - eine definierte Menge wenigstens einer Kontroll-Nukleinsäure, wobei die Kontroll-Nukleinsäure weniger als 50% Sequenzidentität zur Template-Nukleinsäure aufweist und wobei besagte Kontroll-Nukleinsäure mindestens einen bekannten Sequenzbereich besitzt,
   - Primer zur Amplifikation der Template-Nukleinsäure,
   - Primer zur Amplifikation der Kontroll-Nukleinsäure, und
   - Enzyme und Reagenzien geeignet zur Amplifikation von Nukleinsäuren;
Koamplifikation der Template-Nukleinsäure und der Kontroll-Nukleinsäure, wobei die Koamplifikation isothermal erfolgt, wobei die Koamplifikation eine Strand-Displacement-Reaktion umfasst und wobei in besagter Amplifikation Template- und Kontroll-Nukleinsäure im wesentlichen vollständig amplifiziert werden;
Nach Abschluss der Koamplifikation erfolgt die Quantifizierung der amplifizierten Kontroll-Nukleinsäure.

Bei der vorliegenden Erfindung werden also eine oder mehrere exogene Kontroll-Nukleinsäuren dem Reaktionsgemisch zur Amplifikation einer oder mehrerer komplexer Template-Nukleinsäure zugeführt wird. Nach Zugabe von Primem sowie Reagenzien und Enzyme, die zur Amplifikation der komplexen Template-Nukleinsäure und/oder der Kontroll-Nukleinsäure geeignet sind erfolgt eine Koamplifikation der komplexen Template- und Kontroll-Nukleinsäure. Die Kontroll-Nukleinsäure wird also parallel zur komplexen Template-Nukleinsäure in der gleichen Reaktion amplifiziert. Wie stark die Kontroll-Nukleinsäure amplifiziert wird, hängt von der Anwesenheit, der Menge und der Qualität der komplexen Template-Nukleinsäure ab. Somit handelt es sich um eine kompetitive Kontrolle, da die Kontroll-Nukleinsäure und die Template-Nukleinsäure um die Ressourcen, wie z.B. Primer, dNTPs, Polymerase, der Amplifkationsreaktion konkurrieren. Nach erfolgter Koamplifikation wird/werden die amplifizierten Kontroll-Nukleinsäure quantifiziert. Anhand der so bestimmten Quantität der amplifizierten Kontroll-Nukleinsäure lässt sich auf die Ausgangsmenge, bzw. Qualität der komplexen Template-Nukleinsäure rückschließen. Eine geringe Quantität an Kontroll-Nukleinsäure nach der Amplifikation korreliert mit einer ausreichenden Quantität und Qualität an eingesetzter komplexer Template-Nukleinsäure. Entsprechend lässt sich anhand einer größeren Menge an amplifizierten Kontroll-Nukleinsäure auf mangelnde Quantität, bzw. Qualität der eingesetzten komplexen Template-Nukleinsäure schließen. Zudem erlaubt die Bestimmung der relativen Quantität der Kontroll-Nukleinsäure im Gemisch aus Amplifizierten Nukleinsäuren einen Rückschluss auf die Reaktionsbedingungen hinsichtlich pH, Puffer, Salzkonzentration oder Polymeraseaktivität.

Zusätzlich zur ermittelten Menge amplifizierter Kontroll-Nukleinsäure wird die Gesamtmenge amplifizierter Nukleinsäure nach erfolgter Koamplifikation bestimmt. Ist die so bestimmte relative Quantität amplifizierter Kontroll-Nukleinsäure und die Gesamtmenge amplifizierter Nukleinsäure hoch, so wird erfingdungsgemäß mit geeigneten Reaktionsbedingungen für die Amplifikation korreliert. Ist die Gesamtmenge amplifizierter Nukleinsäure niedrig und auch die Quantität an amplifizierter Kontroll-Nukleinsäure so zeigt dies ungeeignete Reaktionsbedingungen für die Amplifikation an.

Das vorliegende Verfahren kann auch verwendet werden, um Reaktionsbedingungen und/oder die Aktivität von Polymerasen zu testen. Dies gilt insbesondere, wenn keine Template-Nukleinsäure sondern ausschließlich die Kontroll-Nukleinsäure eingesetzt wird. Die gemäß der vorliegenden Erfindung verwendete Kontroll-Nukleinsäure besitzt je mindestens einen Bereich definierter Sequenz, mit dem mindestens eine sequenzspezifische Sonden und/oder ein oder mehrere Primer hybridisieren können. Erfindungsgemäß besitzt die Kontroll-Nukleinsäure weniger als 50% Sequenzidentität mit der komplexen Template-Nukleinsäure über die gesamte Länge der Kontroll-Nukleinsäure, bevorzugt weniger als 35%, besonders bevorzugt weniger als 20%. Die Bestimmung von Sequenzidentität zwischen zwei Sequenzen erfolgt erfingdungsgemäß mit dem mathematischen Algorithmus von Karlin und Altschul (1993) Proc. Natl. Acad. Sci. USA 90: 5873-5877.

Weiter umfasst eine lineare Kontroll-Nukleinsäure im Zusammenhang mit der vorliegenden Erfindung eine Länge von mindestens 800 Nukleotide, bevorzugt mehr als 1.000 und weniger als 80.000 Nukleotide, besonders bevorzugt mehr als 5.000 und weniger als 50.000 Nukleotide. Die Kontroll-Nukleinsäure kann natürlichen Ursprungs sein, also aus Organismen, wie z.B. Phagen, Viren, Bakterien oder eukaryotischen Organismen, isoliert. Sie kann aber erfindungsgemäß auch eine nicht natürlich vorkommende Sequenz besitzen. Dem Fachmann ist ersichtlich, dass auch eine Kombination aus natürlich vorkommenden und nicht natürlich vorkommenden Sequenzen als Kontroll-Nukleinsäure dienen kann. Die Kontroll-Nukleinsäure kann aus DNA oder RNA bestehen und sowohl einzel- als auch doppelsträngig vorliegen.

In einer bevorzugten erfindungsgemäßen Ausführungsform wird das Lamda-Phagen-Genom als Kontroll-Nukleinsäure verwendet. Besonders bevorzugt ist die Verwendung eines oder mehrere Fragmente des Lambda-Phagen-Genoms. Fragmente können z.B. durch das Verdauen der Nukleinsäure unter Verwendung von Nukleasen erzeugt werden. Dem Fachmann ist bekannt, dass solche Nukleasen auch sequenzspezifisch Restriktions-Endonukleasen sein können.

In einer anderen bevorzugten erfindungsgemäßen Ausführungsform liegt die Kontroll-Nukleinsäure zirkulär vor. Dem Fachmann ist bekannt, dass solche zirkuläre Kontroll-Nukleinsäure unterschiedlicher Herkunft sein kann. So kann die zirkuläre aus Viren, Phagen, Mikroorganismen (einzellige Organismen) oder auch mehrzelligen Organismen strammen. Eine zirkuläre Kontroll-Nukleinsäure kann erfindungsgemäß auch weniger als 800 nt umfassen. In einer bevorzugten erfindungsgemäßen Ausführungsform umfasst die zirkuläre Kontroll-Nukleinsäure jedoch mehr als 50 nt, besonders bevorzugt mehr als 100 nt. Erfindungsgemäß umfasst die zu analysierende komplexe Template-Nukleinsäure mehr als 10.000 Nukleotide, bevorzugt zwischen 100.000 und 1.000.000 Nukleotide, besonders bevorzugt mehr als 1.000.000 Nukleotide.

Die komplexe Template-Nukleinsäure ist vorzugsweise DNA oder RNA. Sie kann z.B. Nukleinsäure ausgewählt aus der Gruppe von cDNA (complementary DNA), LNA (locked nucleic acid), mRNA (messenger RNA), mtRNA (mitochondriale RNA), rRNA (ribosomale RNA), tRNA (transfer-RNA), nRNA (nucleäre RNA), dsRNA (doppelsträngige RNA), ribozyme, riboswitch, virale RNA, dsDNA (doppelsträngige DNA), ssDNA (einzelsträngige DNA), plasmid DNA, cosmid DNA, chromosomal DNA, viral DNA, mtDNA (mitochondrial DNA), nDNA (nucleäre DNA) sein. Weiter kann die komplexe Template-Nukleinsäure auch die Gesamtheit einer Gruppen von Nukleinsäuren sein, vorzugsweise die Gesamtheit von mRNA, bzw. cDNA (Transkriptom) und/oder die Gesamtheit von DNA (Genom) eines oder mehrere Organismen. Dem Fachmann ist bekannt, dass auch die Gesamtheit von RNA das Genom eines Organismus darstellen kann, dies ist insbesondere bei RNA-Viren der Fall. Die komplexe Nukleinsäure kann auch aus einem oder mehreren Chromosomen bestehen. Die komplexe Template-Nukleinsäure kann fragmentiert oder nicht-fragmentiert vorliegen. Sie kann auch vor der Amplifikation durch enzymatische, physikalische oder chemische Verfahren fragmentiert werden. Ein Bisulfitome ist erfindungsgemäß eine komplexe DNA, wobei nicht methylierte Cytosinbasen durch Verwendung einer Bisulfit-Behandlung in Uracil-Basen umgewandelt wurden.

In einer bevorzugten Ausführungsform des erfindungsgemäßen Verfahrens ist die zu analysierende komplexe Template-Nukleinsäure, Template-Nukleinsäure in einer WGA und/oder WTA und/oder WBA.

Die zu analysierende komplexe Template-Nukleinsäure kann unterschiedlichen Ursprungs sein. Sie kann z.B. aus einem oder mehreren Organismen ausgewählt aus der Gruppe umfassend, Viren, Phagen, Bakterien, Eukaryoten, Pflanzen, Pilze und Tiere isoliert worden sein. Weiter kann die zu analysierende komplexe Template-Nukleinsäure Bestandteil von Proben sein. Solche Proben können ebenfalls unterschiedlicher Herkunft sein. So betrifft das erfindungsgemäße Verfahren auch die Analyse komplexer Template-Nukleinsäure die in Umweltproben enthalten sind.

Im erfindungsgemäßen Verfahren kann auch eine Lyse des die komplexen Template-Nukleinsäure enthaltenden Organismus vor der Amplifikation nötig sein. Unter dem Begriff Lyse wird im Rahmen der vorliegenden Erfindung ein Prozess verstanden, der dazu führt, dass Nukleinsäuren und/oder Proteine aus einem Probenmaterial heraus in die Umgebung abgegeben werden. Dabei kann die Struktur des Probenmaterials zerstört werden, z.B. die Hülle des Probenmaterial aufgelöst werden. Unter dem Begriff "Lyse" wird im Rahmen der vorliegenden Erfindung auch verstanden, dass die komplexe Template-Nukleinsäure aus dem Probenmaterial durch kleine Öffnungen, z.B. Poren etc. in der Hülle des Probenmaterials austreten können, ohne dass dabei die Struktur der Probenmaterials zu zerstören. Beispielsweise können durch Lysereagenzien Poren erzeugt werden. Des Weiteren ist im Rahmen der vorliegenden Erfindung unter dem Begriff "Lyse" zu verstehen, dass Nukleinsäuren und/oder Proteine vom Probenmaterial, das schon strukturell zerstört erscheinen oder kleine Öffnungen aufweisen, durch die Verwendung eines Additivs herausgespült werden können. Durch die Lyse wird ein Lysat erzeugt. Das Lysat kann Probenmaterial verschiedener oder eines einzelnen Organismus enthalt.

Unter einer Amplifikation wird erfingdungsgemäß die Vermehrung einer oder mehrerer Nukleinsäuren um den Faktor 2 oder mehr verstanden. Hierzu kann die Nukleinsäure linear oder exponentiell vermehrt werden. Bei exponentiellen Amplifikationsverfahren wird die Nukleinsäure stärker als um den Faktor 2 vermehrt. Es können auch andere enzymatischen Verfahren zur Anwendung kommen, diese können ausgewählt sein aus der Gruppe umfassend, die "rolling circle amplification" (wie in Liu, et al., "Rolling circle DNA synthesis: Small circular oligonucleotides as efficient templates for DNA polymerases," J. Am. Chem. Soc. 118:1587-1594 (1996) beschrieben), die "isothermal amplification" (wie in Walker, et al., "Strand displacement amplification--an isothermal, in vitro DNA amplification technique," Nucleic Acids Res. 20(7):1691-6 (1992) beschrieben), die "ligase chain reaction" (wie in Landegren, et al., "A Ligase-Mediated Gene Detection Technique," Science 241:1077-1080, 1988, oder in Wiedmann, et al., "Ligase Chain Reaction (LCR)-Overview and Applications," PCR Methods and Applications (Cold Spring Harbor Laboratory Press, Cold Spring Harbor Laboratory, NY, (1994) pp. S51-S64.) beschrieben).

Besagte Amplifkation kann erfingdungsgemäß mit verschiedenen anderen Verfahren und Reaktionen kombiniert werden. Dem Fachmann sind entsprechende Nukleinsäure modifizierenden Verfahren bekannt, z. B. Reverse Transkription, Ligation, Nukleinsäure Auffüllreaktionen, terminale Template-unabhängige Additionen, Verdau mit Restriktionsendonukleasen und/oder die Behandlung mit sogenannten Nicking-Enzymen. Zudem können auch Helicasen und/oder Einzelstrangbindeproteine (z.B. SSB, T4gp32, rec A etc.) zum Einsatz kommen. Eine lineare Amplifikation erreicht man beispielsweise mittels "Rolling-Circle-Amplifikation" (RCA) in Gegenwart von Primern, die auf dem Target Circle nur mit einer spezifischen Sequenz hybridisieren. Eine exponentielle Amplifikation erreicht man beispielsweise über RCA mit Primern, wobei die Primer mit mindestens 2 Bindestellen auf dem Target Circle hybridisieren oder aber mit mindestens einer Bindestelle auf dem Target Circle und mindestens einer Bindestelle auf dem komplementären Strang hybridisieren. Weitere lineare und exponentielle für die vorliegende Erfindung geeignete Amplifikationsverfahren sind dem Fachmann geläufig, wie z.B. MDA oder PCR

Die im Zusammenhand mit der vorliegenden Erfindung verwendete Amplifikationsreaktion ist vorzugsweise eine isothermale Strand-Displacement-Reaktion (wie in Walker, et al., "Strand displacement amplification-an isothermal, in vitro DNA amplification technique," Nucleic Acids Res. 20(7): 1691-6 (1992) beschrieben) zur Koamplifikation von komplexer Template- und Kontroll-Nukleinsäure verwendeten. Strand-Displacement-Reaktion wird hier jede Reaktion verstanden, bei der eine Polymerase verwendet wird, die eine Strand-Displacement Aktivität aufweist, oder bei der eine Reaktionsbedingung verwendet wird, die ein Strand-Displacement ermöglicht. Hierzu zählen z.B. die Strand-Displacement-Amplifikation (SDA) genauso wie die Multiple-Displacement-Amplifikation (MDA) oder die Rolling-Circle-Amplifikation (RCA) sowie alle Unterformen dieser Reaktionen, wie z.B. Restriction-Aided RCA (RCA-RCA) oder MDA mit nested Primern, lineare und exponentielle Strand-Displacement-Reaktionen oder auch Helicase-Dependent Amplifikation (EP 20050112639; EP 20050074804; EP 20050069939; EP 20050069938; Wang G. et al., (2004), DNA amplification method tolerant to sample degradation, Genome Res. Nov;14(11):2357-2366; Milla M.A. et al., (1998), Use of the restriction enzyme AvaI and exo-Bst polymerase in strand displacement amplification, Biotechniques Mar;24(3):392-396; Nagamine K. et al., (2001), Loop-mediated isothermal amplification reaction using a nonde-natured template, Clin Chem. 47(9):1742-1743; Notomi et al (2000), Loop-mediated isothermal amplification of DNA,Nucleic Acids Res.28(12):E63 Lage J.M. et al. (2003), Whole genome analysis of genetic alterations in small DNA samples using hyperbranched strand displacement amplification and array-CGH, Genome Res.13(2):294-307, und Vincent M., Xu Y., Kong H. (2004), Helicase-dependent isothermal DNA amplification, EMBO Rep. 5(8):795-800).

Unter einer isothermalen Reaktion wird erfindungsgemäß eine Reaktion verstanden, die bei nur einer Temperatur durchgeführt wird. Wird die Reaktion vor Beginn (z.B. auf Eis) oder nach Ablauf der Reaktion (z.B. zur Inaktivierung von Reaktionskomponenten oder Enzymen) auf eine andere Temperatur gebracht, wird die Reaktion immer noch isothermal genannt, solange die eigentliche Reaktion bei einer konstanten Temperatur durchgeführt wird. Eine Temperatur wird auch dann als konstant verstanden, wenn die Schwenkung der Temperatur +/- 10°C nicht überschreitet.

Strand-Displacement-Aktivität einer Polymerase bedeutet erfindungsgemäß, dass das eingesetzte Enzym dazu in der Lage ist, einen Nukleinsäure-Doppelstrang in zwei Einzelstränge aufzutrennen. RNA-Polymerasen haben meist eine Strand-Displacement-Aktivität. Bekanntes Beispiel ist die T7-RNA-Polymerase. Weitere Beispiele sind dem Fachmann bekannt. DNA Polymerasen mit Strand-Displacement-Aktivität, die beispielsweise bei der RCA eingesetzt werden können, sind z.B. Holoenzyme oder Teile von Replikasen aus Viren, Prokaryonten, Eukaryonten, oder Archaeen, Phi 29-artige DNA-Polymerasen, die DNA-Polymerase Klenow exo- und die DNA-Polymerase aus *Bacillus stearothermophilus* mit der Bezeichnung Bst exo-. "exo-" bedeutet, dass das entsprechende Enzym keine 5'-3'-Exonukleaseaktivität aufweist. Ein bekannter Vertreter der Phi 29-artigen DNA-Polymerasen ist die DNA-Polymerase aus dem Bakteriophagen Phi 29. Andere Phi 29-artige DNA-Polynerasen kommen z.B. in den Phagen Cp-1, PRD-1, Phi 15, Phi 21, PZE, PZA, Nf, M2Y, B103, SF5, GA-1, Cp-5, Cp-7, PR4, PR5, PR722 und L 17 vor. Weitere geeignete DNA-Polymerasen mit Strand-Displacement-Aktivität sind dem Fachmann bekannt. Alternativ werden unter DNA-Polymerasen mit Strand-Displacement-Aktivität auch solche DNA-Polymerasen ohne Strand-Displacement-Aktivität verstanden, wenn neben einer entsprechenden DNA-Polymerase einen Katalysator verwendet wird, beispielsweise ein Protein oder ein Ribozym, welcher die Trennung eines DNA-Doppelstrangs oder die Stabilisierung von DNA-Einzelsträngen ermöglicht. Zu diesen Proteinen gehören z.B. die Helicasen, SSB-Proteine und Rekombinationsproteine, die als Bestandteil größerer Enzymkomplexe wie z.B. Replikasen enthalten sein können. In diesem Falle erzeugt man mit Komponenten zusätzlich zu der Polymerase eine Polymerase mit Strand-Displacement-Aktivität. Die Polymerasen mit Strand-Displacement Aktivität können hitzelabil oder hitzestabil sein.

In einer bevorzugten Ausführungsform ist die zur Koamplifikation verwendete Polymerase mit Strand-Displacement-Aktivität eine Phi 29-ähnlichen Polymerase, vorzugsweise eine Polymerase aus einem Phagen ausgewählt aus einer Gruppe von Phagen umfassend Phi 29, Cp-1, PRD-1, Phi 15, Phi 21, PZE, PZA, Nf, M2Y, B103, SF5, GA-1, Cp-5, Cp-7, PR4, PR5, PR722 und L 17. Besonders Bevorzugt ist die Verwendung der Polymerase aus dem Phagen Phi 29.

Es ist für den Fachmann ersichtlich, dass auch die Verwendung von Mischungen von zwei oder mehr Polymerasen mit Strand-Displacement-Aktivität möglich ist. Weiter kann auch eine oder mehrere Polymerasen mit Strand-Displacement-Aktivität mit einer oder mehreren Polymerase ohne Strand-Displacement-Aktivität kombiniert werden.

In einer bevorzugten Ausführungsform des erfindungsgemäßen Verfahrens werden die zur Amplifikation der komplexen Template-Nukleinsäure eingesetzten Primer auch zur Amplifikation der Kontroll-Nukleinsäure verwendet.

Unter Primer im Sinne der vorliegenden Erfindung wird ein Molekül verstanden, das als Startstelle für ein Enzym mit Nukleinsäure-Polymerase-Aktivität dient. Dieser Primer kann ein Protein, eine Nukleinsäure oder ein anderes Molekül sein, das sich dem Fachmann als Polymerase-Startstelle geeignet erweist. Dieses Molekül kann durch intermolekulare aber auch durch intramolekulare Wechselwirkung als Startstelle dienen. Im Falle von Nukleinsäure-Primern müssen diese nicht, können aber über ihre gesamte Länge mit der Template-Nukleinsäure hybridisieren.

Besonders bevorzugt ist die Verwendung von Random-Primern für die Koamplifikation von Kontroll- und komplexer Template-Nukleinsäure, d.h. ein Primergemisch umfassend mehrere unterschiedliche Primer zufälliger Sequenz.

Es können neben Random-Primern auch andere Primer zur Amplifikation der Template-, bzw. Kontroll-Nukleinsäure zur Anwendung kommen. So können auch degenerierte und/oder sequenzspezifische Primer zu Amplifikation der Kontroll- und/oder der Template-Nukleinsäure verwendet werden.

Die zur Amplifikation verwendeten Primer umfassen 4 bis 25 Basen, bevorzugt zwischen 5 bis 15 Basen, besonders bevorzugt 6 bis 10 Basen.

Die Quantifizierung der amplifizierten Kontroll-Nukleinsäure im erfindungsgemäßen Verfahren erfolgt über den mindestens einen bekannten Sequenzbereich. Bevorzugt ist eine Quantifizierung mittels quantitativer (Real-Time) PCR (qRT-PCR) durch Amplifizierung eines Teils oder der gesamten Kontroll-Nukleinsäure unter Verwendung von sequenzspezifischen, mit dem mindestens einen bekannten Sequenzbereich der Kontroll-Nukleinsäure hybridisierenden Primern.

In einer bevorzugten Ausführungsform wird Lamda-Phagen-DNA oder Fragmente davon als Kontroll-Nukleinsäure verwendet. In dieser Ausführungsform des erfindungsgemäßen Verfahrens erfolgt die Quantifizierung der Kontroll-Nukleinsäure durch qRT-PCR vorzugsweise mittels sequenzspezifischer Primer. In einem besonders bevorzugten Ausführungsform Primer der Sequenz SEQ ID NO. 1 bzw. SEQ ID NO. 2.

Die DNA-Polymerase, die während der quantitativen (Real-Time) PCR verwendet wird ist vorzugsweise eine Polymerase aus einem thermophilen Organismus oder ist eine thermostabile Polymerase oder ist eine Polymerase ausgewählt aus der Gruppe von *Thermus thermophilus* (Tth) DNA Polymerase, *Thermus acquaticus* (Taq) DNA Polymerase, *Thermotoga maritima* (Tma) DNA Polymerase, *Thermococcus litoralis* (Tli) DNA Polymerase, *Pyrococcus furiosus* (Pfu) DNA Polymerase, *Pyrococcus woesei* (Pwo) DNA Polymerase, *Pyr ococcus kodakaraensis* KOD DNA Polymerase, *Thermus filiformis* (Tfi) DNA Polymerase, *Sulfolobus solfataricus* Dpo4 DNA Polymerase, *Thermus pacificus* (Tpac) DNA Polymerase, *Thermus eggertsonii* (Teg) DNA Polymerase, *Thermus brockianus* (Tbr) und *Thermus flavus* (Tfl) DNA Polymerase.

Im Falle das RNA, beispielsweise mRNA, muss die RNA in DNA revers transkribiert werden. Dies geschieht mit einem Enzym mit Reverser Transkriptase-Aktivität. Solche Enzyme können z.B. Reverse Transkriptasen aus Viren, Bakterien, Archae-Bakterien und Eukaryonten, insbesondere aus thermostabilen Organismen sein. Hierzu zählen z.B. auch Enzyme aus Introns, Retrotransposons oder Retroviren. Ein Enzym mit reverser Transkriptaseaktivität ist erfindungsgemäß ein Enzym, welches in der Lage ist, an einer Ribonukleinsäure am 3'-Ende eines an die Ribonukleinsäure-hybridisierten Desoxyoligonukleotides oder Ribooligonukleotides bei geeigneten Pufferbedingungen Desoxyribonukleotide komplementär einzubauen. Dies umfasst zum einen Enzyme, die natürlicherweise diese Funktion aufweisen aber auch Enzyme, die eine solche Funktion erst durch Verränderung ihrer Gensequenz wie z.B. Mutagenese oder durch entsprechende Pufferbedingungen erhalten.

Bevorzugt ist das Enzym mit reverser Transkriptaseaktivität ein Enzym, welches ausgewählt ist aus der Gruppe umfassend HIV reverse Transkriptase, M-MLV reverse Transkriptase, EAIV reverse Transkriptase, AMV reverse Transkriptase, *Thermus thermophilus* DNA Polymerase I, M-MLV RNAse H, Superscript, Superscript II, Superscript III, Monstersript (Epicentre), Omniscript, Sensiscript Reverse Transkriptase (Qiagen), ThermoScript und Thermo-X (beide Invitrogen). Erfindungsgemäß können auch Enzyme verwendet werden, die erst nach einer Modifikation der Gensequenz als Enzym reverse Transkriptaseaktivität aufweisen. Es kann auch eine Reverse Transkriptaseaktivität verwendet werden, die eine erhöhte Fehlergenauigkeit aufweist. Beispielhaft sei hier z.B. AccuScript reverse Transcriptase (Stratagene) erwähnt. Es ist für den Fachmann ersichtlich, dass auch die Verwendung von Mischungen von zwei oder mehr Enzymen mit reverser Transkriptaseaktivität möglich ist.

Dem Fachmann ist bekannt, dass die meisten Enzyme mit reverser Transkriptaseaktivität ein divalentes Ion benötigen. Somit liegt einer bevorzugten Ausführungsform bei jenen Enzymen, die ein divalentes Ion benötigen, ein divalentes Ion vor. Bevorzugt sind Mg2+, Mn2+.

Bevorzugte Kombinationen von Enzymen sind HIV reverse Transkriptase oder M-MLV reverse Transkriptase oder EAIV reverse Transkriptase oder AMV reverse Transkriptase oder *Thermus thermophilus* DNA Polymerase I oder M-MLV RNAse H minus, Superscript, Superscript II, Superscript III oder Monstersript (Epicentre) oder Omniscript Reverse Transkriptase (Qiagen) oder Sensiscript Reverse Transkriptase (Qiagen), ThermoScript, Thermo-X (beide Invitrogen) oder eine Mischung von zwei oder mehr Enzymen mit reverser Transkriptaseaktivität und Poly-(A)-Polymerase aus *Escherichia coli.* Außerdem HIV reverse Transkriptase oder M-MLV reverse Transkriptase oder EAIV reverse Transkriptase oder AMV reverse Transkriptase oder *Thermus thermophilus* DNA Polymerase I oder M-MLV RNAse H minus, Superscript, Superscript II, Superscript III oder Monstersript (Epicentre) oder Omniscript Reverse Transkriptase (Qiagen) oder Sensiscript Reverse Transkriptase (Qiagen), ThermoScript, Thermo-X (beide Invitrogen) oder eine Mischung von zwei oder mehr Enzymen mit reverser Transkriptaseaktivität und Poly-(A)-Polymerase aus Hefe.

In der qRT-PCR können fluoreszenzmarkierte Primer und/oder Sonden verwendet werden, z.B. LightCycler-Sonden (Roche), TaqMan Sonden (Roche), Molecular beacons, Scorpion-Primer, Sunrise-Primer, LUX-Primer oder Amplifluor-Primer. Sonden und/oder Primer können z.B. kovalent oder nicht-kovalent gebundene Fluoreszenzfarbstoffe beispielsweise Fluoresceinisothiocyanate (FITC), 6-Carboxyfluorescein (FAM), Xanthen, Rhodamine, ,6-Carboxy-2',4',7',4,7-hexachlorofluorescein (HEX), 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine 110; Coumarine, wie Umbelliferone, Benzimide, wie Hoechst 33258; Phenanthridine, wie Texas Red, Ethidiumbromide, Acridinfarbstoffe, Carbazolfarbstoffe, Phenoxazinefarbstoffe, Porphyrinfarbstoffe, Polymethinfarbstoffe, Cyaninfarbstoffe, wie Cy3, Cy5, Cy7, SYBR-Green, BODIPY-Farbstoffe, Quinolinfarbstoffe und Alexa Farbstoffe enthalten.

Dem Fachmann ist bekannt, dass in qRT-PCR auch unabhängig von Primer und Sonden, Doppelstrang-spezifische Fluoreszenzfarbstoffe, z.B. Ethidiumbromid, SYBR Green, PicoGreen, RiboGreen etc. verwendet werden können.

Dem Fachmann sind die geeigneten Bedingungen für eine quantitative PCR oder eine quantitative Reverse Transkriptions-PCR bekannt. Dies betrifft z.B. das Primer-Design, die Wahl von geeigneten Prozessierungstemperaturen (Reverse Transkription, Denaturierung, Primer Annealing, Elongation), die Zahl der PCR-Zyklen, die Pufferbedingungen. Reverse Transkription und die Auswertung der quantitativen (Real-Time) PCR.

Erfindungsgemäß werden niedrige in der qRT-PCR ermittelte Ct (threshold Cycle) Werte für ein oder mehrere Amplikon(s) der Kontroll-Nukleinsäure mit einer niedrigen Qualität und/oder niedrigen Quantität der komplexen Templat-Nukleinsäure korreliert. Entsprechen stehen hohe Ct-Werte für eine hohe Qualität und/oder hohe Quantität der komplexen Template-Nukleinsäure.

In einer bevorzugten erfindungsgemäßen Ausführungsform wird die Qualität und/oder Quantität der zu amplifizierenden komplexen Template-Nukleinsäure über den Delta-Ct-Wert ermittelt. Besagter Delta-Ct-Wert wird berechnet aus der Differenz zwischen dem ermittelten Ct-Wert für die Kontroll-Nukleinsäure nach Koamplifikation mit der komplexen Template-Nukleinsäure und dem Ct-Wert für die Kontroll-Nukleinsäure in einer Kontrollreaktion. Die Kontrollreaktion zeichnet sich dadurch aus, dass keine komplexe Template-Nukleinsäure zugefügt wird. Jedoch bleiben die übrigen Bestandteile sowie die Reaktionsbedingungen unverändert. Je höher der Delta-Ct-Wert der Amplikons der Kontroll-Nukleinsäure nach der Amplifikation der komplexen Nukleinsäure ist, desto höher ist die Qualität der für die Amplifikation verwendeten komplexen Nukleinsäure.

Die vorliegende Erfindung betrifft auch ein, wie in Anspruch 13 definierter, Kit zur Durchführung eines erfindungsgemäßen Verfahrens wie oben beschrieben umfassend:
- Random Primer oder sequenzspezifische Primer zur Amplifikation der Kontroll-Nukleinsäure und der komplexen Template-Nukleinsäure,
- eine oder mehrere Kontroll-Nukleinsäuren, wobei die Kontroll-Nukleinsäuren je mindestens einen bekannten Sequenzbereich besitzen;
- eine DNA-Polymerase mit Strand-Displacement-Aktivität,
   und ein oder mehrere Prirnerpaare und/oder sequenzspezifische Sonden, wobei die Primer und/oder die sequenzspezifische Sonde im bekannten Sequenzbereiche der Kontroll-Nukleinsäure hybridisieren können und geeignet sind die Kontroll-Nukleinsäure zu quantifizieren
   Optional kann das erfindungsgemäße Kit weitere Komponenten enthalten, z.B.
- eine Pufferlösung oder eine Pufferstammlösung,
- dNTPs (vorzugsweise als Lösung einer Mischung aus dCTP, dATP, dGTP und dTTP (z.B. jeweils 5 mM, "dNTP Mix"),
- eine thermostabile DNA-Polymerase,
- mindestens einen Doppelstrang-spezifischen Fluoreszenzfarbstoff,
- optional eine Pufferlösung oder eine Pufferstammlösung,
   und/oder - Enzyme und Reagenzien zur Durchführung einer qRT-PCR.

Ein erfindungsgemäßes Kit kann auch eine Arbeitsanweisung zur Durchführung des oben beschriebenen Verfahrens enthalten.

Die Höhe der Ct-Werte der Amplikons der Kontroll-Nukleinsäure hängt, neben der Quantität und/oder Qualität der komplexen Template-Nukleinsäure, in entscheidendem Maße von der eingesetzten Menge an Kontroll-Nukleinsäure und den Reaktionsbedingungen der Amplifikationsreaktion ab. Um nun WGA, WTA und /oder WBA vergleichen zu können, die nicht gleichzeitig durchgeführt wurden, ist es wünschenswert standardisierte Bedingungen herzustellen. Dies ist jedoch nicht immer in vollem Umfang möglich. Um nun Reaktionen vergleichen zu können, die zu unterschiedlicher Zeit und/oder an unterschiedlichen Orten mit unter Umständen unterschiedlichen Bedingungen durchgeführt wurden, werden beispielsweise interne Standards der Reaktionen benötigt. Dem Fachmann ist ersichtlich, dass die erfindungsgemäßen Verfahren und/oder Kits zur Verwendung als solche Standards in WGA und/oder WTA und/oder WBA geeignet sind.

Die Erfindung umfasst somit auch die Verwendung eines oben genannten Verfahrens oder des erfindungsgemäßen Kits zur Standardisierung von WGA und/oder WTA und/oder WBA. Besonders bevorzugt ist erfingdungsgemäß die Verwendung von ermittelten Delta-Ct-Werten zur Standardisierung von WGA und/oder WTA und/oder WBA.

### Sequenzen

| **SEQ ID NO.** | **Sequenz** | **Verwendung** |
|---|---|---|
| SEQ ID NO. 1 | 5'-GAGACGCTGGAGTACAAACG-3' | Forward Primer für den Marker Standard 1 der Kontroll-Nukleinsäure |
| SEQ ID NO. 2 | 5'-CCAGCGGATTATCGCCATACTG-3' | Reverse Primer für den Marker Standard 1 der Kontroll-Nukleinsäure |
| SEQ ID NO. 3 | 5'-TGCTCCCTGTCCCATCTG-3' | Forward Primer für den Marker 699 |
| SEQ ID NO. 4 | 5'-AGACAGTATGCCTTTATTTCACCC-3' | Reverse Primer für den Marker 699 |
| SEQ ID NO. 5 | 5'-GTCTTTAGCTGCTGAGGAAATG-3' | Forward Primer für den Marker 1004 |
| SEQ ID NO. 6 | 5'-AGCAGAATTCTGCACATGACG-3' | Reverse Primer für den Marker 1004 |
| SEQ ID NO. 7 | 5'-AGTGCTAATGTCATGTCTCTT-3' | Forward Primer für den Marker HexA |
| SEQ ID NO. 8 | 5'-GCTGAGAGGAGTGGAAGCT-3' | Reverse Primer für den Marker HexA |
| SEQ ID NO. 9 | 5'-AGTGCTAATGTCATGTCTCTT-3' | Forward Primer für den Marker HexG |
| SEQ ID NO. 10 | 5'-GCTGAGAGGAGTGGAAGCC-3' | Reverse Primer für den Marker HexG |

### Beschreibung der Zeichnungen

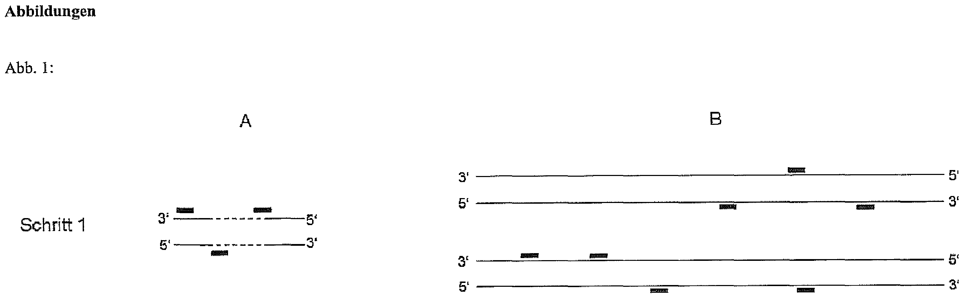

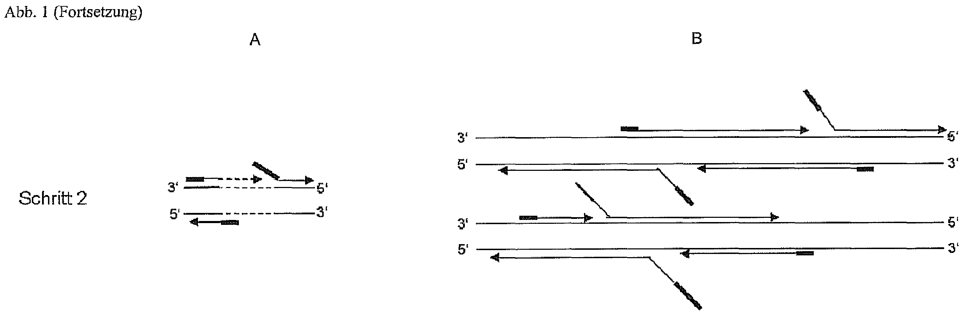

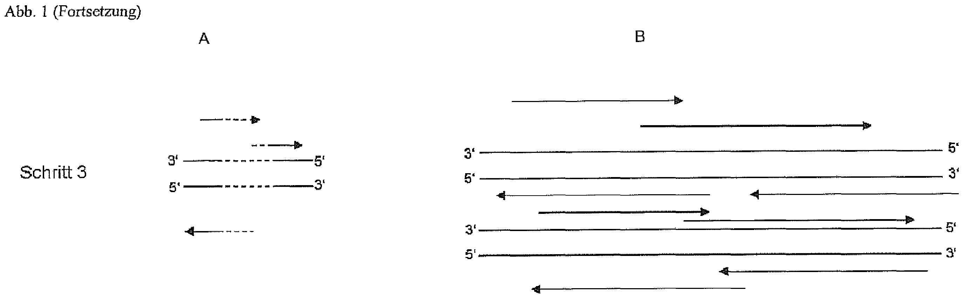

Abbildung 1: Schematische Darstellung des beanspruchten Verfahrens. In Schritt 1 werden Kontroll-Nukleinsäure (A), komplexe Template-Nukleinsäure (B) und Primer bereitgestellt. In Schritt 2 erfolgt die Koamplifikation von Kontroll- und komplexer Template-Nukleinsäure. Nach Abschluss der Koamplifikation wird in Schritt 3 die Menge an amplifizierter Kontroll-Nukleinsäure über den bekannten Sequenzbereich (gestrichelte Linie) bestimmt.

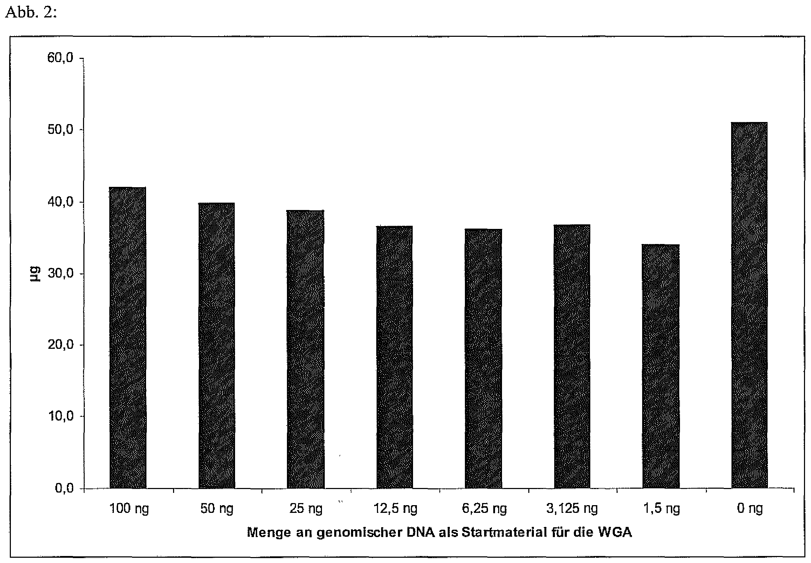

Abbildung 2: Ausbeute an Gesamt-DNA nach erfolgter Whole-Genome-Amplifikation: für die WGA wurde eine Mischung aus Kontroll-DNA (jeweils 20 pg) und unterschiedlichen Mengen an Template-Nukleinsäure (0-100 ng) eingesetzt.

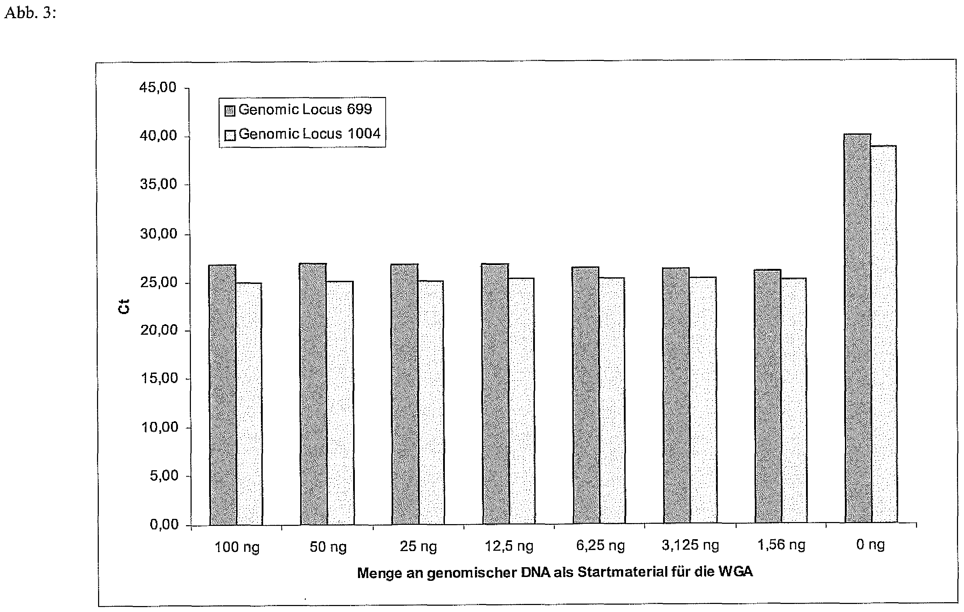

Abbildung 3: Repräsentanz der Template-Nukleinsäure Marker 699 und 1004 in 10 ng WGA DNA. Auf der Y-Achse wurde der Ct-Wert aus der Real-Time-PCR Analyse dargestellt. Es bestand keine Korrelation zwischen dem Ct-Wert und der Menge an Template-Nukleinsäure für die WGA Reaktion.

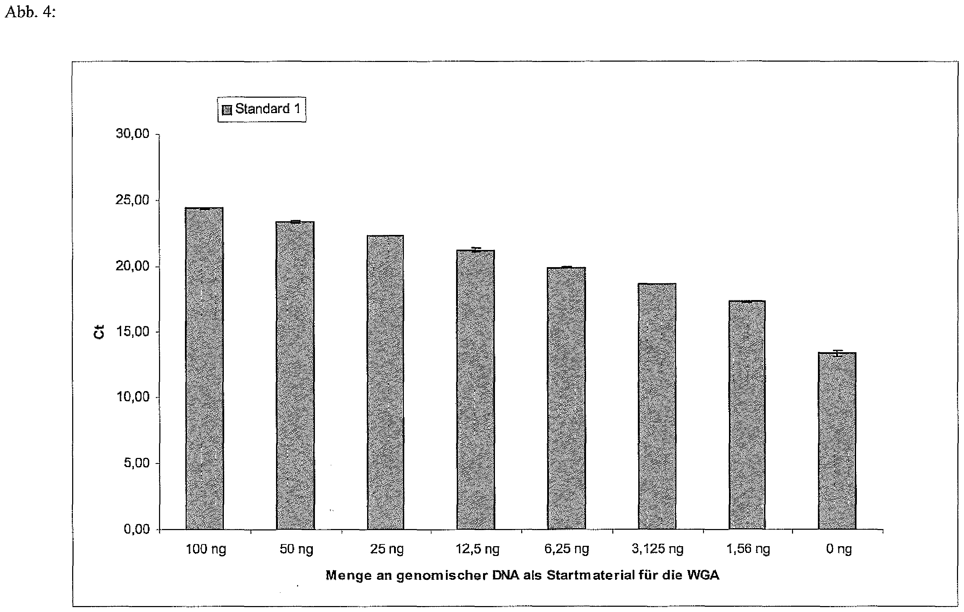

Abbildung 4: Repräsentanz des Kontroll-Nukleinsäure Markers Standard 1 in 10 ng WGA DNA. Auf der Y-Achse wurde der Ct-Wert aus der Real-Time PCR Analyse dargestellt. Es bestand eine deutliche Korrelation zwischen dem Ct-Wert und der Menge an Template-Nukleinsäure für die WGA Reaktion.

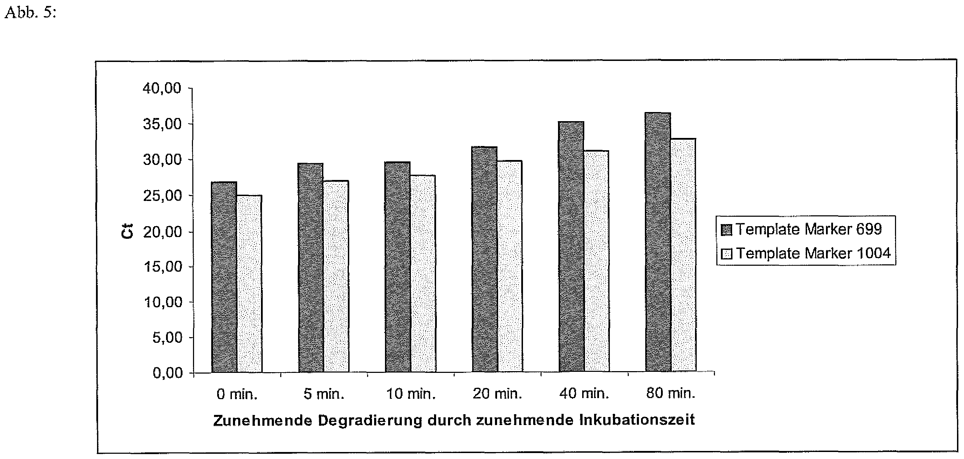

Abbildung 5: Repräsentanz der Template-Nukleinsäure Marker 699 und 1004 in 10 ng WGA DNA. Auf der Y-Achse wurde der Ct-Wert aus der Real-Time-PCR Analyse dargestellt. Auf der X-Achse wurde die fortschreitende Degradierung der template-Nukleinsäure durch Zunahme der Inkubationszeit der Template-Nukleinsäure mit *Hae*III dargestellt.

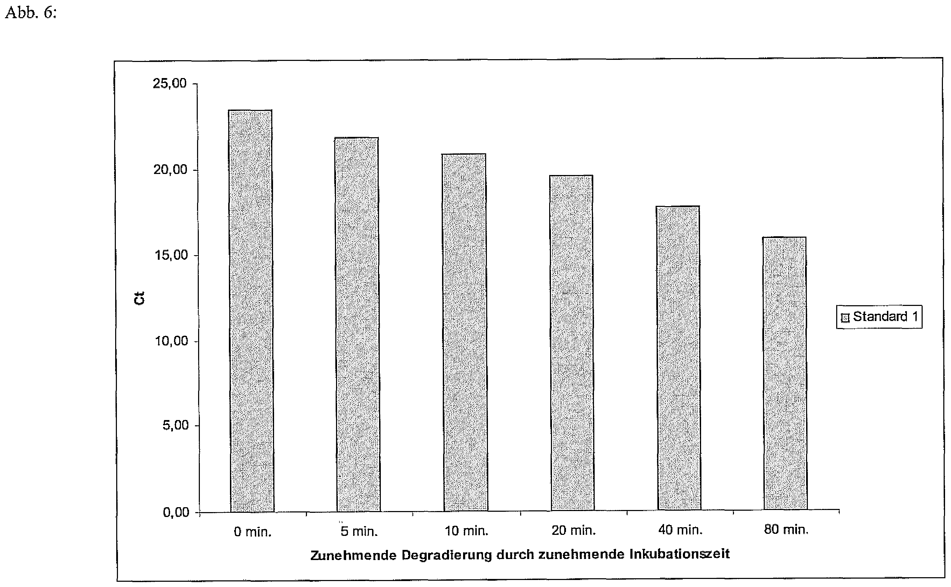

Abbildung 6: Repräsentanz des Kontroll-Nukleinsäure Marker Standard 1 in 10 ng WGA DNA. Auf der Y-Achse wurde der Ct-Wert aus der Real-Time PCR Analyse dargestellt. Auf der X-Achse wurde die fortschreitende Degradierung der Template-Nukleinsäure durch Zunahme der Inkubationszeit der Template-Nukleinsäure mit *Hae*III dargestellt.

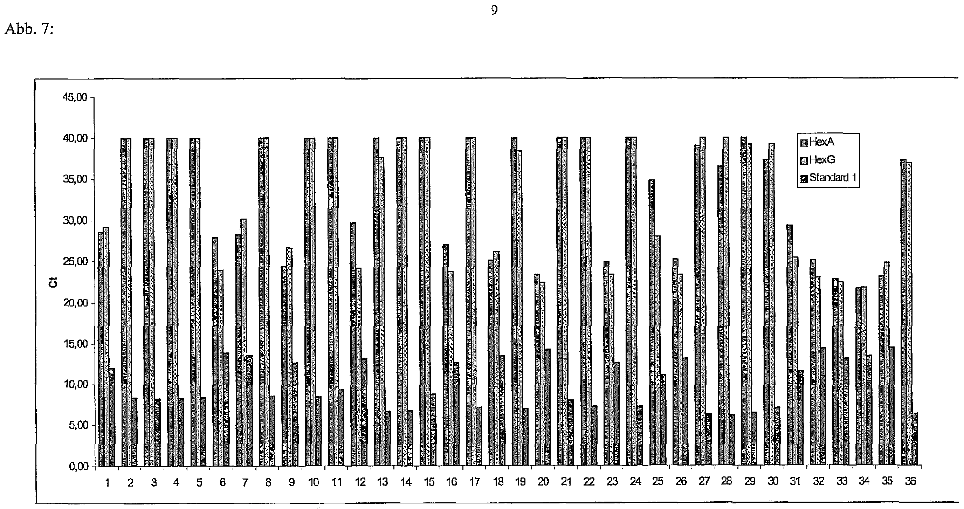

Abbildung 7: 36 WGA Reaktionen wurden durchgeführt unwissend, ob das Reaktionsgefäß eine Zelle (also Template-Nukleinsäure) enthielt oder nicht. Repräsentanz des Kontroll-Nukleinsäure Marker Standard 1 ist in der Real-Time-PCR immer dann hoch (niedriger Ct-Wert von < 10), wenn keine Template-Nukleinsäure im Reaktionsgefäß war (Ct-Wert ∼40 bei Marker HexA und HexG). Konnte man jedoch Hex A und Hex G nachweisen (Ct-Wert - 22 bis 30), dann war der Ct-Wert des Markers Standard 1 >10. Somit kann über die Kontroll-Nukleinsäure und der anschließenden PCR die Anwesenheit der Template-Nukleinsäure nachgewiesen werden.

### Beispiele

### Beispiel 1:

Verfahren zur Bestimmung der Menge an Template-Nukleinsäure, die für die WGA unter Verwendung von Kontroll-Nukleinsäuren bei WGA Reaktionen eingesetzt wurde.

Hintergrund:: Die Repräsentanz der Kontroll-Nukleinsäure in der WGA-DNA nach der Whole-Genome-Amplifikation von Template-Nukleinsäure und Kontroll-Nukleinsäure kann auf die Menge der ursprünglich eingesetzten Template-Nukleinsäure hinweisen.

Es wurden unterschiedliche Mengen an humane genomischer DNA (0; 1,5; 3,125; 6,25; 12,5; 25; 50 oder 100 ng) in eine Whole-Genome-Amplifikation (WGA) Reaktion eingesetzt. Die WGA Reaktion wurde mit REPLI-g Reagenzien nach Angaben des Herstellers durchgeführt (Qiagen). Zu den einzelnen WGA Reaktion wurden 20 pg einer Lambda DNA als Kontroll-Nukleinsäure zugefügt, wobei die Kontroll-Nukleinsäure im Gegensatz zur Template-Nukleinsäure nicht denaturiert wurde. Nach der WGA Reaktion wurde die Konzentration und Ausbeute an Gesamt-DNA mithilfe des PicoGreen Reagenz (siehe REPLI-g Handbuch) bestimmt (Abbildung 1).

Die Repräsentanz der Kontroll-Nukleinsäure und der Template-Nukleinsäure wurde mittels quantitativer Real-Time-PCR untersucht. Dazu wurden 10 ng der entstandenen Gesamt-DNA in einer quantitativen Real-Time-PCR (QuantiTect Real-time PCR SYBR Green; Qiagen) eingesetzt. Dabei wird der Ct-Wert (Threshold cycle) der Kontroll-Nukleinsäure als Maß für die repräsentative Amplifikation der komplexen Template-DNA angegeben. Der Ct-Wert gibt den PCR Zyklus an, an dem die Fluoreszenz zum ersten Mal über der Basis-Fluoreszenz nachgewiesen werden kann. Eine niedrige Repräsentanz der Marker würde zu hohen Ct-Werten, eine hohe Repräsentanz zu niedrigen Ct-Werten führen. Bleibt die Repräsentanz gleich bei verschiedenen Ansätzen, so ist keine Verränderung im Ct-Wert zu beobachten. Ein Ct-Wert von ungefähr 40 Zyklen gibt an, dass der untersuchte Marker nicht in dem untersuchten Ansatz vorhanden ist.

Für die Untersuchung der Repräsentanz an komplexer Template-Nukleinsäure nach erfolgter WGA-Reaktion wurden die in der eingesetzten Template-DNA enthaltenen Marker 699 und Marker 1004 in quantitativen Real-Time PCR Reaktionen quantifiziert. Für die Untersuchung der Repräsentanz der Kontroll-Nukleinsäure nach der WGA wurde der darin enthaltende Marker Standard 1 verwendet. Auch hier wurde jeweils 10 ng der amplifizierten Gesamt-DNA eingesetzt. Als Real-Time PCR Reagenzien wurden die QuantiTect Real-Time PCR SYBR Green Reagenzien (QIAGEN) nach Herstellerangaben verwendet. Als Primer für den Marker 699 wurden Oligonukleotide der SEQ ID NO. 3 und SEQ NO. 4 verwendet. Als Primer für den Marker 1004 wurden Oligonukleotide der SEQ ID NO. 5 und SEQ ID NO. 6 verwendet. Als Primer für den Marker Standard 1 der Kontroll-Nukleinsäure wurden Oligonukleotide der SEQ ID NO. 1 und SEQ ID NO. 2 verwendet.

Ergebnis: Die Messung der Ausbeute an Gesamt-DNA nach den erfolgten Whole-Genome-Amplifikations-Reaktionen ergab eine nur geringe Abnahme an amplifizierter Gesamt-DNA abhängig von der eingesetzten Menge an Template-Nukleinsäure (1,56 ng bis 100 ng; Abbildung 2).

Für die Untersuchung der Repräsentanz der Template-Nukleinsäure-Marker 699 und 1004 in der amplifizierten Gesamt-DNA wurden für die verschiedenen WGA Ansätze jeweils 10 ng in die qRT-PCR eingesetzt. Die Untersuchung ergab keinerlei Korrelation des Ct-Wertes mit der eingesetzten Startmaterialmenge von komplexer Template-Nukleinsäure in die WGA. Der Ct-Wert blieb während der Real-Time PCR Analyse trotz unterschiedlicher Mengen an Template-Nukleinsäure für die WGA konstant (Abbildung 3).

Bei Verwendung von 10 ng an WGA Gesamt-DNA für die Untersuchung der Repräsentanz der Kontroll-Nukleinsäure in einer Real-Time PCR wurde jedoch eine strenge Korrelation des Ct-Wertes mit der ursprünglichen Menge an Startmenge der Template-Nukleinsäure für die WGA-Reaktion festegestellt: Je geringer die Startmenge an Template-Nukleinsäure für die WGA war desto niedriger war auch der Ct-Werts des Markers Standard 1, der die Kontroll-Nukleinsäure repräsentiert (Abbildung 4).

### Beispiel 2:

Verfahren zur Bestimmung der Qualität/Integrität der komplexen Template-Nukleinsäure, die für die WGA, unter Verwendung einer Kontroll-Nukleinsäure, eingesetzt wurde.

Hintergrund: Die Repräsentanz der Kontroll-Nukleinsäure in der Gesamt-DNA nach der WGA von komplexer Template-Nukleinsäure und Kontroll-Nukleinsäure kann auf die Qualität/Integrität der ursprünglich eingesetzten komplexen Template-Nukleinsäure hinweisen.

Durchführung: Genomische DNA wird durch einen partiellen Verdau mit der Restriktions-Endonuklease *Hae*III geschädigt. Hierzu wird 20 µg humane genomische DNA in einem 200 µl Ansatz mit 1,9 U *Hae*III für 0, 5, 10, 20, 40 und 80 Minuten bei 37°C inkubiert. Die hierdurch entstehende, zeitlich zunehmende Spaltung der DNA durch *Hae*III repräsentieren unterschiedliche Degradierungsgrade der komplexen Template-Nukleinsäure und stellt somit unterschiedliche Qualitäts-/Integritätsstufen der komplexen Template-Nukleinsäure dar. 10 ng der so gewonnenen Template-Nukleinsäure unterschiedlichen Degradierungsgrades wurde zusammen mit 20 pg Kontroll-DNA in einer WGA eingesetzt. Die WGA Reaktion wurde mit REPLI-g Reagentien nach dem REPLI-g Herstellerangaben durchgeführt (Qiagen), wobei die Kontroll-Nukleinsäure im Gegensatz zur Template-Nukleinsäure nicht denaturiert wurde. Nach erfolgter WGA wurde die Konzentration und Ausbeute der DNA mithilfe des PicoGreen Reagenz (siehe REPLI-g Handbuch) bestimmt.

Die Repräsentanz der Kontroll-DNA und der komplexen Template-DNA wurde in qRT-PCR untersucht. Dazu wurden 10 ng der in der WGA entstandenen Gesamt-DNA in einer Real-Time PCR (QuantiTect Real-time PCR SYBR Green Reagenzien, Qiagen) eingesetzt. Für die Untersuchung der Repräsentanz der komplexen Template-Nukleinsäure nach erfolgter WGA wurden die Marker 699 und Marker 1004 in der WGA amplifizierten DNA in qRT-PCR verwendet. Für die Untersuchung der Repräsentanz der Kontroll-Nukleinsäure nach der WGA wurde der Marker Standard 1 herangezogen. Hier wurden ebenfalls jeweils 10 ng der amplifizierten Gesamt-DNA eingesetzt. Als qRT-PCR Reagenzien wurden die QuantiTect Real-Time PCR SYBR Green Reagenzien (Qiagen) eingesetzt. Als Primer für den Marker 699 wurden Oligonukleotide der SEQ ID NO. 2 und SEQ ID NO. 3 verwendet. Als Primer für den Marker 1004 wurden Oligonukleotide der SEQ ID NO. 4 und SEQ ID NO. 5 verwendet. Als Primer für den Marker Standard 1 der Kontroll-Nukleinsäure wurden Oligonukleotide der SEQ ID NO. 1 und SEQ ID NO. 2 verwendet.

Ergebnis: Es ist bekannt, dass bei zunehmender Verschlechterung der Qualität der Template-Nukleinsäure die Repräsentanz der Template-Nukleinsäure nach WGA immer kleiner wird. Dies war auch hier zu beobachten: Mit zunehmender Degradierung (Fragmentierung) der Template-Nukleinsäure, angezeigt durch die zunehmende Inkubationszeit (min) der Template-Nukleinsäure mit *Hae*III, erhöht sich der Ct-Wert in der Real-Time Analyse von 10 ng WGA DNA (siehe Abbildung 5).
Für die Repräsentanz der Kontroll-Nukleinsäure nach erfolgter WGA ergibt sich eine eindeutige Korrelation mit der Zunahme an Fragmentierung der ursprünglich eingesetzten komplexen Template-Nukleinsäure (siehe Abbildung 5). Die Zunahme der Repräsentanz der Kontroll-Nukleinsäure - angezeigt durch Verringerung des Ct-Wertes des Markers Standard 1 - korreliert mit der Fragmentierung (Zunahme der *Hae*III Inkubationszeit) und der Annahme der Repräsentanz der Template-Nukleinsäure Marker (699 und 1004) (vgl. Abbildung 4 und 6).

### Beispiel 3:

Verfahren zur Bestimmung der Anwesenheit der Template-Nukleinsäure unter Verwendung einer Kontroll-Nukleinsäure bei WGA Reaktionen.

Hintergrund: Die Repräsentanz der Kontroll-Nukleinsäure in der Gesalnt-DNA nach erfolgter WGA von komplexer Template-Nukleinsäure und Kontroll-Nukleinsäure kann auf die prinzipielle Anwesenheit der ursprünglich eingesetzten Template-Nukleinsäure hinweisen.

Durchführung: Eine Zellkultur wird so verdünnt, dass ungefähr nur jedes zweites Reaktiongefäß eine Zelle enthält. Jedem Reaktionsgefäß werden 20 pg Kontroll-Nukleinsäure (Lambda-DNA) hinzugefügt. Vor der WGA wird eine Lyse der Zellen durchgeführt. Die Lyse wird dem REPLI-g Handbuch entsprechend durchgeführt. Anschließend wird eine Whole-Genome-Amplifikation mit REPLI-g Reagentien (QIAGEN) dem REPLI-g Handbuch entsprechend durchgeführt. Nach der WGA Reaktion wurde die Konzentration und Ausbeute der DNA mithilfe des PicoGreen Reagenz (siehe REPLI-g Handbuch) bestimmt.

Die Repräsentanz der Kontroll-DNA und der komplexen Template-Nukleinsäure wurde in qRT-PCR untersucht. Dazu wurden 10 ng der in der WGA entstandenen Gesamt-DNA in einer qRT-PCR (QuantiTect Real-time PCR SYBR Green Reagenzien; Qiagen) eingesetzt. Für die Untersuchung der Repräsentanz der komplexen Template-Nukleinsäure nach der WGA wurden die beiden Hexokinase Allele HexA und HexG in der WGA amplifizierten DNA in qRT-PCR Reaktionen analysiert. Für die Untersuchung der Repräsentanz der Kontroll-Nukleinsäure nach der WGA wurde der Marker Standard 1 verwendet. Auch hier wurde jeweils 10 ng der amplifizierten Gesamt-DNA eingesetzt. Als qRT-PCR Reagenzien wurden die QuantiTect Real-time PCR SYBR Green Reagenzien (Qiagen) eingesetzt.

Ergebnis: Wann immer eine Zelle und somit auch die komplexe Template-Nukleinsäure im Reaktionsgefäß vorhanden war, erhält man einen Ct-Wert für die Allele HexA und HexG der deutlich kleiner ist als 40. Diese Anwesenheit korreliert deutlich mit einem Ct-Wert von > 10 für den Marker Standard 1, der die Amplifikation der Kontroll-Nukleinsäure repräsentiert. Im Mittel wurde ein Wert von 13,05 (+/- 0,95) erreicht. In den Fällen, in denen keine Zelle (also keine Template-Nukleinsäure) vorhanden war, wurde im Mittel ein Ct-Wert von 7,5 (+/-0,95) für den Marker Standard 1 erreicht. Somit ist ein Ct-Wert > 10 (Marker Standard 1) indikativ für die Anwesenheit der Template-Nukleinsäure, wohingegen ein Ct-Wert < 10 die Abwesenheit der Template-Nukleinsäure indiziert (Abbildung 7).

## Patentansprüche

1. Verfahren zur quantitativen und qualitativen Analyse einer oder mehrerer zu amplifizierender komplexer Template-Nukleinsäuren in einer Probe umfassend die folgenden Schritte:
a) Bereitstellung eines Reaktionsgemisches umfassend
- die zu amplifizierende komplexe Template- Nukleinsäure
- eine definierte Menge wenigstens einer Kontroll-Nukleinsäure, wobei die Kontroll-Nukleinsäure weniger als 50% Sequenzidentität zur Template-Nukleinsäure aufweist und wobei besagte Kontroll-Nukleinsäure mindestens einen bekannten Sequenzbereich besitzt,
- Primer zur Amplifikation der Template- Nukleinsäure,
- Primer zur Amplifikation der Kontroll- Nukleinsäure,
- Enzyme und Reagenzien geeignet zur Amplifikation von Nukleinsäuren;
b) Koamplifikation der Template-Nukleinsäure und der Kontroll- Nukleinsäure, wobei die Koamplifikation isothermal erfolgt, wobei die Koamplifikation eine Strand-Displacement-Reaktion umfasst und wobei in besagter Amplifikation Template- und Kontroll-Nukleinsäure im wesentlichen vollständig amplifüziert werden;
c) Quantifizierung der amplifizierten Kontroll-Nukleinsäure, wobei die Quantifizierung nach Abschluss der Koamplifikation aus Schritt b) erfolgt, wobei zusätzlich die Gesamt-Nukleinsäuremenge nach Abschluss der Koamplifikation quantifiziert wird;
wobei die Primer zur Amplifikation der Templat-Nukleinsäure und der Kontrollnukleinsäure identisch sind; wobei die Kontrollnukleinsäure eine exogene Nukleinsäure ist;
wobei die Quantität der amplifizierten Kontroll- Nukleinsäure und die Quantität an Gesamt-Nukleinsäure mit einer erfolgten Amplifikation der komplexen Template-Nukleinsäure korreliert werden;
wobei die komplexe Template-Nukleinsäure mehr als 10.000 Nukleotide umfasst.

2. Verfahren nach Anspruch 1, wobei die Kontroll-Nukleinsäure linear ist.

3. Verfahren nach Anspruch 2, wobei die lineare Kontroll-Nukleinsäure mehr als 800 Nukleotide umfasst.

4. Verfahren nach Anspruch 1, wobei die Kontroll-Nukleinsäure zirkulär ist.

5. Verfahren nach Anspruch 1 bis 4, wobei die komplexe Template-Nukleinsäure(n) ausgewählt ist aus der Gruppe umfassend DNA und RNA.

6. Verfahren nach Anspruch 1 bis 5, wobei die komplexe Template-Nukleinsäure ein oder mehrere Genome und/oder ein oder mehrere Transkriptome und/oder ein oder mehrere Bisulfitome umfasst.

7. Verfahren nach Anspruch 1, wobei die Polymerase eine Phi 29-ähnliche Polymerase.

8. Verfahren nach Anspruch 1 bis 7, wobei Random-Primer zur Koamplifikation verwendet werden.

9. Verfahren nach Anspruch 1 bis 8, wobei die Quantifizierung der amplifizierten Kontroll-Nukleinsäure in Schritt c) über den mindestens einen bekannten Sequenzbereich erfolgt.

10. Verfahren nach Anspruch 9, wobei die Quantifizierung der amplifizierten Kontroll-Nukleinsäure unter Verwendung des mindestens einen bekannten Sequenzbereichs mittels mindestens einer spezifischen Sonde und/oder mittels Amplifikation über sequenzspezifische Primer erfolgt.

11. Kit zur Durchführung eines Verfahrens nach Anspruch 1 bis 10 umfassend:
- Random Primer
- eine oder mehrere Kontroll-Nukleinsäuren, wobei die Kontroll-Nukleinsäuren je mindestens einen bekannten Sequenzbereich besitzen;
- Eine Polymerase mit Strand-Displacement- Aktivität
- mindestens ein Primerpaar für den bekannten Sequenzbereich der Kontroll-Nukleinsäuren und/oder mindestens eine sequenzspezifische Sonde für den bekannten Sequenzbereich der Kontroll-Nukleinsäuren.

12. Verwendung eines Verfahrens nach Anspruch 1 bis 10 oder eines Kits nach Anspruch 11 zur Standardisierung von Whole-Genome- und/oder Whole-Transkriptome- und/oder einer Whole-Bisulfitome-Amplifikationen.

## Claims

1. A method for quantitative and qualitative analysis of one or more complex template nucleic acids to be amplified in a sample, comprising the following steps:
a) providing a reaction mixture comprising
- the complex template nucleic acid to be amplified,
- a specified amount of at least one control nucleic acid, wherein the control nucleic acid has less than 50% sequence identity to the template nucleic acid and wherein said control nucleic acid has at least one region of known sequence,
- primers for amplifying the template nucleic acid,
- primers for amplifying the control nucleic acid,
- enzymes and reagents suited for the amplification of nucleic acids;
b) co-amplification of the template nucleic acid and of the control nucleic acid, wherein the co-amplification takes place isothermally, wherein the co-amplification comprises a strand displacement reaction and wherein the template and control nucleic acids are essentially completely amplified in said amplification;
c) quantification of the amplified control nucleic acid, wherein the quantification is performed after completion of the co-amplification of step b), wherein the total amount of nucleic acid is in addition quantified after completion of the co-amplification;
wherein the primers for the amplification of the template nucleic acid and the control nucleic acid are identical; wherein the control nucleic acid is an exogenous nucleic acid; wherein the amount of the amplified control nucleic acid and the total amount of nucleic acid is correlated with an amplification of the complex template nucleic acid that has taken place;
wherein the complex template nucleic acid comprises more than 10,000 nucleotides.

2. The method according to claim 1, wherein the control nucleic acid is linear.

3. The method according to claim 2, wherein the linear control nucleic acid comprises more than 800 nucleotides.

4. The method according to claim 1, wherein the control nucleic acid is circular.

5. The method according to claims 1 to 4, wherein the complex template nucleic acid(s) is selected from the group comprising DNA and RNA.

6. The method according to claims 1 to 5, wherein the complex template nucleic acid comprises one or several genomes and/or one or several transcriptomes and/or one or several bisulfitomes.

7. The method according to claim 1, wherein the polymerase is a Phi 29-like polymerase.

8. The method according to claims 1 to 7, wherein random primers are used for the co-amplification.

9. The method according to claims 1 to 8, wherein the quantification of the amplified control nucleic acid of step c) is performed on the at least one region of known sequence.

10. The method according to claim 9, wherein the quantification of the amplified control nucleic acid is performed by using the at least one region of known sequence by means of at least one specific probe and/or by means of amplification by sequence-specific primers.

11. Kit to carry out the method of claims 1 to 10 comprising:
- random primers
- one or several control nucleic acids, wherein the control nucleic acids each have at least one region of known sequence;
- a polymerase with strand-displacement activity
- at least one primer pair for the region of known sequence of the control nucleic acid and/or at least one sequence-specific probe for the region of known sequence of the control nucleic acid.

12. Use of a method according to claims 1 to 10 or of a kit according to claim 11 for the standardisation of whole genome and/or of whole transcriptome and/or of whole bisulfitome amplification.

## Revendications

1. Procédé pour l'analyse quantitative et qualitative d'un ou plusieurs acides nucléiques complexes de matrice à amplifier dans un échantillon comprenant les étapes :
a) fourniture d'un mélange réactionnel comprenant
- l'acide nucléique complexe de matrice à amplifier,
- une quantité définie d'au moins un acide nucléique témoin, où l'acide nucléique témoin a une identité de séquence avec l'acide nucléique de matrice de moins de 50% et où ledit acide nucléique témoin possède au moins une région de séquence connue,
- des amorces pour l'amplification de l'acide nucléique de matrice,
- des amorce pour l'amplification de l'acide nucléique témoin,
- des enzymes et des réactifs adaptés pour l'amplification d'acides nucléiques ;
b) co-amplification de l'acide nucléique de matrice et de l'acide nucléique témoin, où la co-amplification est effectuée de manière isotherme, où la co-amplification comprend une réaction de déplacement de brin et où l'acide nucléique de matrice et l'acide nucléique témoin sont essentiellement entièrement amplifiés dans ladite amplification ;
c) quantification de l'acide nucléique témoin amplifié, où la quantification est effectuée après l'achèvement de la co-amplification de l'étape b), où en outre la quantité d'acide nucléique totale est quantifiée après l'achèvement de la co-amplification ;
où les amorces pour l'amplification de l'acide nucléique de matrice et de l'acide nucléique témoin sont identiques ; où l'acide nucléique témoin est un acide nucléique exogène ;
où la quantité de l'acide nucléique témoin amplifié et la quantité d'acide nucléique totale sont corrélées à une amplification de l'acide nucléique de matrice qui a eue lieu ;
où l'acide nucléique de matrice comprend plus de 10.000 nucléotides.

2. Le procédé selon la revendication 1, dans lequel l'acide nucléique témoin est linéaire.

3. Le procédé selon la revendication 2, dans lequel l'acide nucléique témoin comprend plus de 800 nucléotides.

4. Le procédé selon la revendication 1, dans lequel l'acide nucléique témoin est circulaire.

5. Le procédé selon l'une quelconque des revendications 1 à 4, dans lequel l'acide nucléique complexe de matrice est choisi du groupe comprenant l'ADN et l'ARN.

6. Le procédé selon l'une quelconque des revendications 1 à 5, dans lequel l'acide nucléique complexe the matrice comprend un ou plusieurs génomes et/ou un ou plusieurs transcriptomes et/ou un ou plusieurs bisulfitomes.

7. Le procédé selon la revendication 1, dans lequel la polymérase est semblable à la polymérase Phi 29.

8. Le procédé selon l'une quelconque des revendications 1 à 7, dans lequel des amorces aléatoires sont utilisées pour la co-amplification.

9. Le procédé selon l'une quelconque des revendications 1 à 8, dans lequel la quantification de l'acide nucléique témoin amplifié dans l'étape c) est effectuée dans au moins une région de séquence connue.

10. Le procédé selon la revendication 9, dans lequel la quantification de l'acide nucléique témoin amplifié est effectuée en utilisant la au moins une région de séquence connue au moyen d'au moins une sonde spécifique et/ou au moyens d'une amplification avec des amorces séquences-spécifiques.

11. Kit pour effectuer le procédé selon l'une quelconque des revendications 1 à 10 comprenant :
- des amorces aléatoires
- un ou plusieurs acides nucléiques témoin, où les acides nucléiques témoin ont chacun au moins une région de séquence connue ;
- une polymérase avec activité de déplacement de brin
- au moins une paire d'amorces pour la région de séquence connue des acides nucléiques témoin et/ou au moins une sonde séquence spécifique pour la région de séquence connue des acides nucléiques témoin.

12. Utilisation d'un procédé selon l'une quelconque des revendications 1 à 10 ou d'un kit selon la revendication 11 pour la standardisation d'amplification du génome entier et/ou du transcriptome entier et/ou du bisulfitome entier.
